# EUROPEAN PATENT SPECIFICATION

(11) **EP 3 981 460 B1**
(45) Date of publication and mention of the grant of the patent: **07.05.2025**
(21) Application number: 21198911.6
(22) Date of filing: 24.09.2021
(51) Int. Cl.: A61M 25/00, A61M 25/01, A61M 25/04

(54) **SELF-SUSTAINABLE URINARY CATHETER**
SELBSTHALTENDER HARNKATHETER
CATHÉTER URINAIRE AUTONOME

(30) Priority: 28.09.2020 BR 102020019762; 18.11.2020 BR 132020023524
(43) Date of publication of application: 13.04.2022
(73) Proprietor: Vilar Correia Lima, Salvador, Cep: 52060-000 (BR); De Oliveira Vilar, Fabio, Cep: 52060-000 (BR)
(72) Inventor: Vilar Correia Lima, Salvador, Cep: 52060-000 (BR); De Oliveira Vilar, Fabio, Cep: 52060-000 (BR)
(74) Representative: Fezzardi, Antonio

(56) References cited:
- EP-A1- 0 479 935
- WO-A1-92/19192
- BR-A2- 102014 021 737
- US-A- 5 707 367

## Description

### FIELD OF APPLICATION

This invention patent application provides a urinary catheter, which encompasses the field of medical and hospital use devices and items.

### INTRODUCTION

This invention patent application provides a permanent, removable and self-sustainable urinary catheter, notably developed to be used on living male and female patients affected by urethral dysfunction, incontinence and chronic urinary retention, among other secondary causes that affect bladder function, in order to provide bladder voiding in a natural manner, simulating the voiding physiology.

As used herein, the terms "permanent, removable" must be understood in relation to the fact that the urinary catheter according to the present invention can be installed on site even for a long time (for example 6 months), and then be removed.

This patent application further incorporates a chuck used for installing the catheter in the patient, which becomes an external chuck and may be used in combination or in replacement of the internal chuck previously developed, as required.

### STATE OF THE ART

The permanent or intermittent bladder catheterization and use of diapers have been the standard solution up to now for patients suffering bladder voiding disorders, either from permanent urine loss, insufficient or lack of voiding.

The absence or decrease of urinary strength at any stage of life renders the bladder incapable of storing urine, causing atrophy of the detrusor muscle structure and decrease, loss or increase of the autonomic nervous system stimulation.

Patients with reduction or loss of urinary strength require mechanisms to reinforce it in order to stimulate the neuromuscular system of the bladder and enable proper urine storage; on the other hand, patients that are unable to void require mechanisms to enable such process.

The simplest way of permanent bladder voiding is the use of a Foley catheter, which comprises an inflatable balloon connected to a collection bag which is emptied at suitable intervals.

Although the bladder is properly voided, its formal structure, the manufacturing material, as well as the inconvenience of using an inflatable balloon, make this solution less popular.

In order to provide improvements to the market of medical and hospital devices and items, the applicant of this application, active in the market and concerned with the good quality of products provided to the consumer market in said area of medical and hospital devices and items, has already filed a patent application No. BR 10 2014 021737 1 before INPI [*Brazil National Institute of Industrial Property*] on 09/02/2014, under the title: "PERMANENT, REMOVABLE AND SELF-SUSTAINABLE URINARY CATHETER", which describes, in summary, a tubular body 5 made of medical grade silicone with a smooth surface, with the final portion of its proximal end 4 closed and the rounded end 4A which includes an orifice 4A'; the proximal end 4 of the tubular 5 features orifices 6 that are connected to a longitudinal channel 7 which traverses the tubular body 5 of the catheter 1 from the extremity of the proximal end 4 to the lumen 8 located in the connector 10; the tubular body 5 includes two discs: a fixed proximal 2 and the other mobile distal 3, both including, respectively, protrusions 2A and 3A.

Said permanent, removable and self-sustainable urinary catheter may be adjusted to the anatomy of the patient and includes a tubular body 5 in which two discs are installed, one proximal (fixed) 2 and another distal (mobile) 3; the distal end 4 of the tubular body 5 of the catheter 1 includes orifices 6 which are connected to a longitudinal channel 7 that crosses the tubular body 5. The catheter includes a connector 10 which may be closed by a corresponding lid 11.

Further, US 5707367 A, WO 92/19192 A1 and EP 0479935 A disclose urinary catheter with three discs; however they do not disclose two sliding discs.

### ISSUES WITH THE STATE OF THE ART

The outlet orifices of the catheter included in the state of the art end up being insufficient in specific cases in which elimination of crystals produced by the patient's kidneys.

Another inconvenience noted resides in the difficulty in placing the catheter through the patient's bladder and, in addition, the lack of ergonomics of said catheter is also seen as an inconvenience, for cases in which the patient is a wheelchair user, as said individuals are usually unable to reach and handle the device properly. On the other hand, the inclusion of a single distal disc may cause sliding of the catheter body, thus leading to loss of urine.

Another inconvenience resides in unwanted movement of the discs during installation in the patient.

### OBJECTIVES OF THE INVENTION

The objective of this patent application is to address the aforementioned needs and provide patients with a urinary catheter with greater comfort enabled by compact dimensions and perfect fitting to the genitourinary anatomy, as well as providing a disposable chuck that greatly helps in positioning said catheter and, lastly, providing proper support to wheelchair users by offering a model with dimensions that facilitate reaching and proper handling by said wheelchair user.

Another objective of this patent application is to provide an external chuck that allows installation of the catheter with no movement of the discs during the procedure, and allows the physician to remove the external chuck and adjust disc positions as required, after the introduction of the catheter.

The external chuck has lower production costs, is of simpler design, more efficient, and greatly facilitates the installation process of the urinary catheter in the patient.

The subject matter of the invention is defined in the appended claims.

### BRIEF DESCRIPTION OF THE OBJECT

In this way, continuing the studies and researches of said permanent, removable and self-sustainable urinary catheter, the applicant, in order to provide even more benefits to the consumer market, therefore, seeks to implement the object of the aforementioned patent application, said application that was registered on behalf of the same aforementioned applicant which, due to the considerable amount of researches and studies, has taken notice of the need of introducing some innovations to the previously developed catheter, in order to allow use for a greater group of people and in a more comfortable, efficient and safe manner.

In face of the particular aspects of the previously presented state of the art, the catheter provided herein was developed, which is manufactured in medical grade silicone and includes three discs with a central protrusion, one of said discs being a fixed proximal which, attached to the bladder neck, promotes occlusion, and two distal sliding discs which, when fitted to the female external meatus or to the male perineal urethrostomy, or even through any other access, provides full occlusion and subsequent continence. The second mobile disc provides increased safety to the first and prevents such issues.

This catheter is defined as a tubular body with six holes on its proximal end, which is 2.5 cm long.

The tubular body is manufactured in medical grade silicone, flexible and transparent, with a smooth surface and a rounded and closed tip (on its proximal end), and features a small 1 mm diameter orifice to allow passage of a guide wire, which may be required in some situations to facilitate introduction.

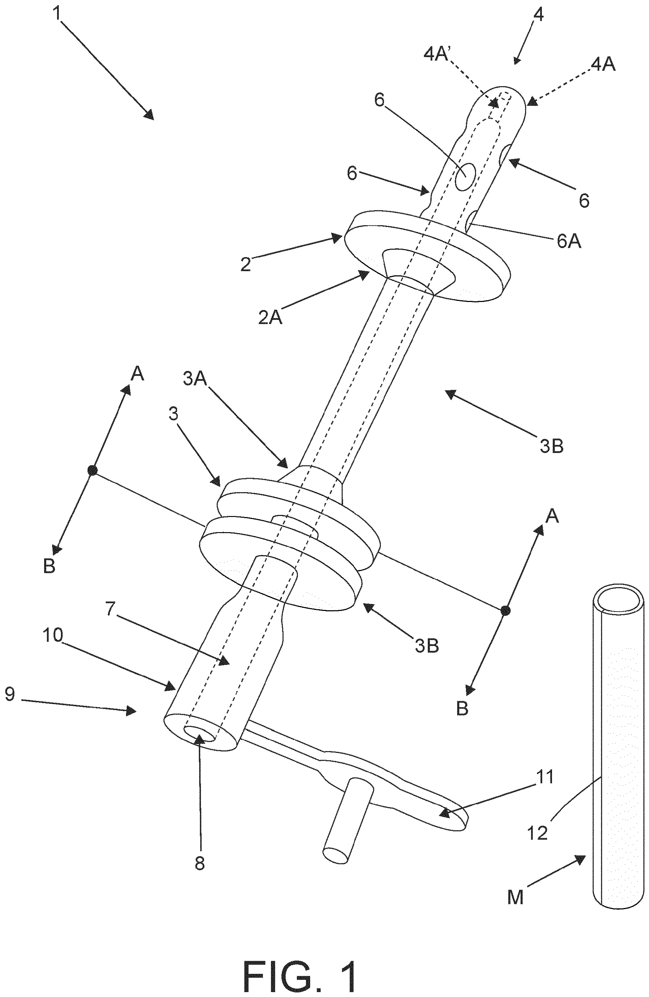

A connector with a lid is provided on the distal end, including an opening and closing mechanism which enables bladder voiding when desired. (Figures 1, 2 and 3) .

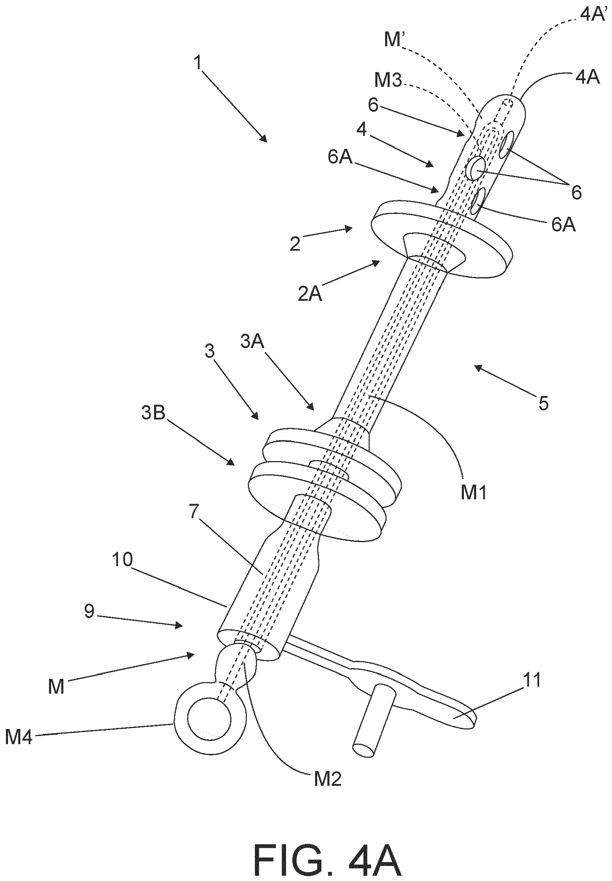

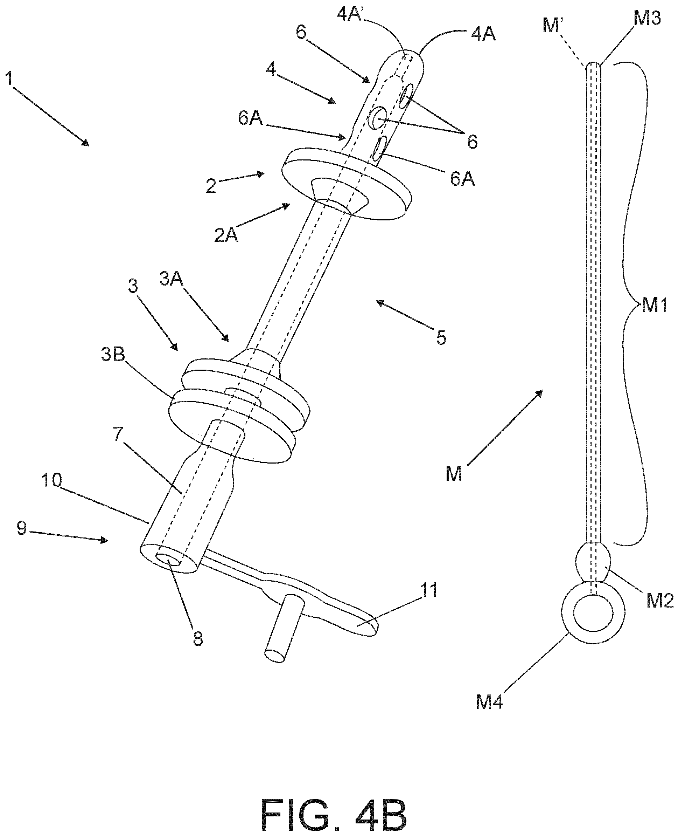

The catheter also includes a disposable chuck made of polypropylene (Figures 4A and 4B) which must allow the introduction of a catheter through the patient's bladder. It comes in two sizes to allow for safe introduction of the bladder in different sizes.

This chuck includes a channel with a 1mm diameter opening along its entire length, in order to enable the potential use of a guide wire, as previously mentioned.

The innovations of this patent application are the reduced and adjustable size, and perfect fitting to the genitourinary anatomy, in addition to a second distal disc.

There is also a special size catheter with a 15 cm extension to be used in wheelchair patients which are unable to reach and properly handle the device. This chuck is part of the package that comes with the specific probe and is also made of polypropylene.

The external chuck is disposable, and consists in a polypropylene tubular element, including a longitudinal opening that extends for the entire length of the chuck, which is already assembled to the catheter.

### DETAILED DESCRIPTION OF THE DRAWINGS

In order to supplement this patent description and to attain better understanding of this invention patent application, and according to a practical, preferred embodiment hereof, a set of drawings is attached hereto, which in an exemplary but not limiting manner, the following aspects are represented:
Figure 1 shows a perspective view of a permanent, removable and self-sustainable urinary catheter, described herein with an indication of possible movement of adjustment of the distal sliding discs, with said movement indicated by the arrows "A"-"B", where branch "A" of the "A"-"B" arrow indicates sliding of the distal discs towards the proximal end of the catheter, while branch "B" of the same arrow "A"-"B" indicates sliding of the distal discs towards the distal end of the catheter and, laterally, it shows a perspective view of the external chuck M;
Figure 2 shows a side view of the catheter portrayed in figure 1, in which readings/distances are indicated for progression towards the distal sliding discs and the orifices present in the proximal end of the tubular body are evidenced;
Figure 2A shows an expanded and perspective detail of the proximal end of the tubular body of this catheter, showing the six orifices;
Figure 3 shows the catheter present in figure 1, inserted through the urethra of a patient and reaching the bladder;
Figure 3A illustrates a similar view to the previous one, but showing the position of one of the distal rings; and
Figures 4A and 4B show, respectively, the catheter described herein, including the chuck and the aforementioned catheter with said chuck on the side, in order to enable introduction of the catheter through the patient's urethra.
Figure 5 illustrates a view of the external chuck applied to the urinary catheter;
Figures 6A to 6D illustrate side views of the urinary catheter proposed herein with emphasis on the coupling between the external chuck and the catheter;
Figures 7A and 7B illustrate the urinary catheter installed in the bladder with the use of the external chuck and removal of the external chuck after installation of the catheter.

### DETAILED DESCRIPTION OF THE INVENTION

The catheter, object of this invention patent application, is indicated in general by reference number 1 and is manufactured in medical grade silicone, including three discs, each one with a central protrusion, in which a fixed proximal disc 2 is coupled to the bladder neck CV and promotes occlusion, said fixed proximal disc 2 including its protrusion indicated by reference 2A; a distal disc 3 with its protrusion indicated by reference 3A and a distal disc 3B, being said distal discs 3, 3B sliding and adjustable to the female external meatus or to the male perineal urethrostomy in order to provide full occlusion of the patient's urethra U and promoting continence.

Protrusions 2A and 3A of discs 2 and 3 may feature a substantially conical format, required for perfect fixing of the catheter to the bladder neck CV and to the external meatus ME of the urethra in female patients, or to the perineal urethrostomy in male patients.

The proximal end 4 of the tubular body 5 of the catheter 1 includes six orifices indicated by numerical references 6 and 6A, through which the volume of urine contained in the patient's bladder B may be drained through a longitudinal channel 7 which crosses the tubular body 5 from the region of orifices 6 and 6A up to the lumen 8 located on the distal end 9.

The inclusion of six orifices in total and, more particularly, regarding the two orifices 6A closest to the proximal disc 2, particularly in diametrically opposed positions to each other, is key to prevent accumulation of crystals generated by urine, which may obstruct the catheter, especially if the device exceeds the time for replacement.

The proximal end 4 is closed and features a rounded tip indicated by reference 4A, in order to allow passage of the aforementioned guide wire (not mentioned), which is not part of the claimed object of this invention.

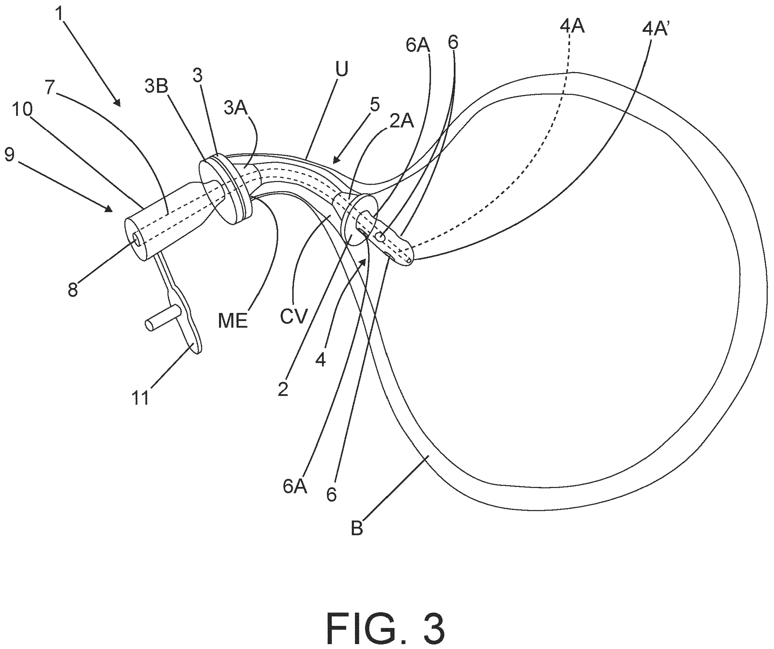

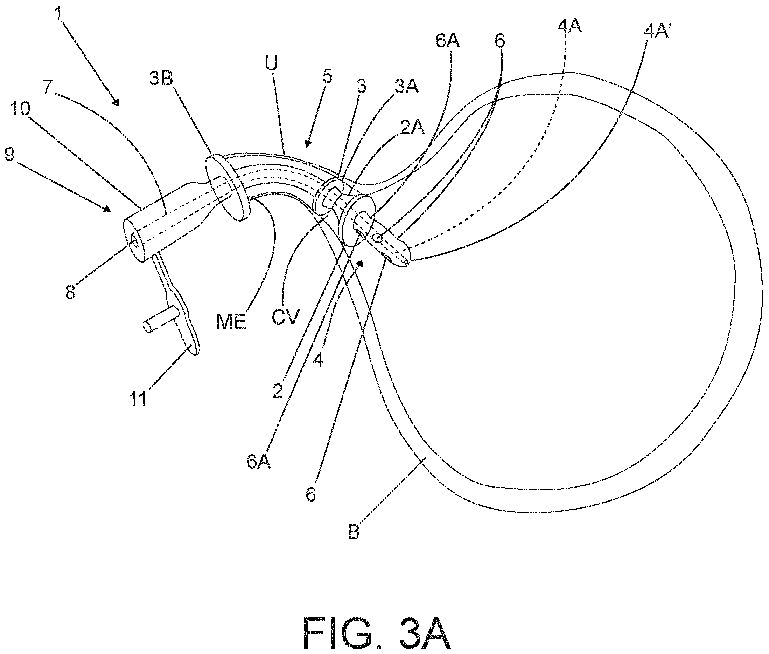

The distal end 9 of the catheter 1, on the other hand, includes a connector 10 with a lid 11 including an opening and closing mechanism which allows voiding the bladder B when desired, such as understood through visualization of figures 3 and 3A.

The catheter 1 also includes a chuck M (such as shown in Figures 4A and 4B) which should allow the introduction of the catheter 1 through the patient's urethra U.

The chuck M features a rod-shaped portion M1, which is dimensioned to be introduced along the tubular body 5 of the catheter 1; an enlarged distal portion M2 which matches its end, when touching the connector end 10, and its final point M3 reaches the closed proximal end 4 of the urinary catheter 1; and a handle portion M4, through which the chuck M is handled.

The chuck M features a channel M' for its entire length, through which a guide wire (not shown) is eventually used in the placement procedure of the catheter 1.

It should be noted that orifice 4A', incorporated to the proximal end 4 of the catheter 1 is included in full alignment with the channel M' provided in the entire chuck M.

The state of the art to be considered useful to the understanding of this invention would derive from the introduction of catheter 1 through the urethra U up to the bladder neck CV, for female patients.

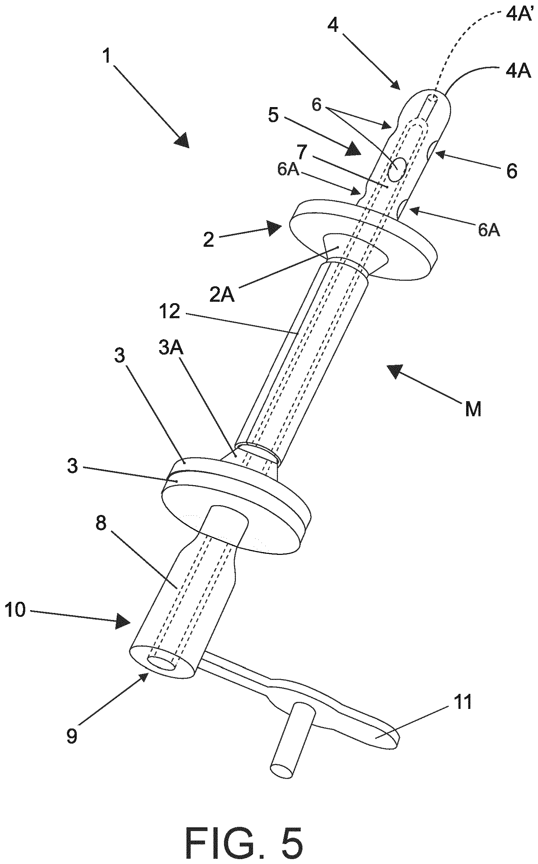

For male patients that require catheter use, it must be introduced through a minor perineal urethrostomy, such as shown in figure 5.

The guide wire mentioned in this document is an accessory and is not part of this invention, as it is used eventually, in other words, in more complex situations that require easier insertion conditions for the catheter.

The catheter addressed herein is indicated, in general, by reference number 1, whereas the three discs included with the catheter (discs 2, 3 and 3B) are malleable and present no difficulty to this procedure.

One disc (proximal disc 2) shall remain in the internal portion of the bladder neck CV (proximal), therefore, inside the bladder B, and the other two distal discs (3 and 3B) to the external meatus of the urethra ME or to the male perineal region (Figure 3A).

The sliding of disc 3 through the catheter 1 allows for adjustment related to the extension of the urethra U, with such adjustment shown regarding the movement shown by arrow "A"-"B" such as featured in figure 1.

This mechanism is worked through traction applied by the person skilled in the art when introducing the catheter 1, after it is attached to the bladder neck CV (disc 2) and the external meatus ME of the urethra (disc 3) through the respective protrusions 2A and 3A present in discs 2 and 3 or the male perineal region.

The catheter 1 described herein may be removed or replaced at any time, and may remain for periods up to six to eight months due to the material used in its manufacturing and reduced dimensions.

The catheter 1 is made in two gauges (13 and 15Fr), for use in children, and four gauges (17, 19, 21, and 23Fr) for use in adults.

The full length of this catheter 1 is 7cm for child models and 8cm for adult models. A 15cm model is also available to be used in patients with special needs, such as, for example, wheelchair users.

In both cases, both the gauge and the catheter size 1 may vary according to anatomy characteristics of the patient.

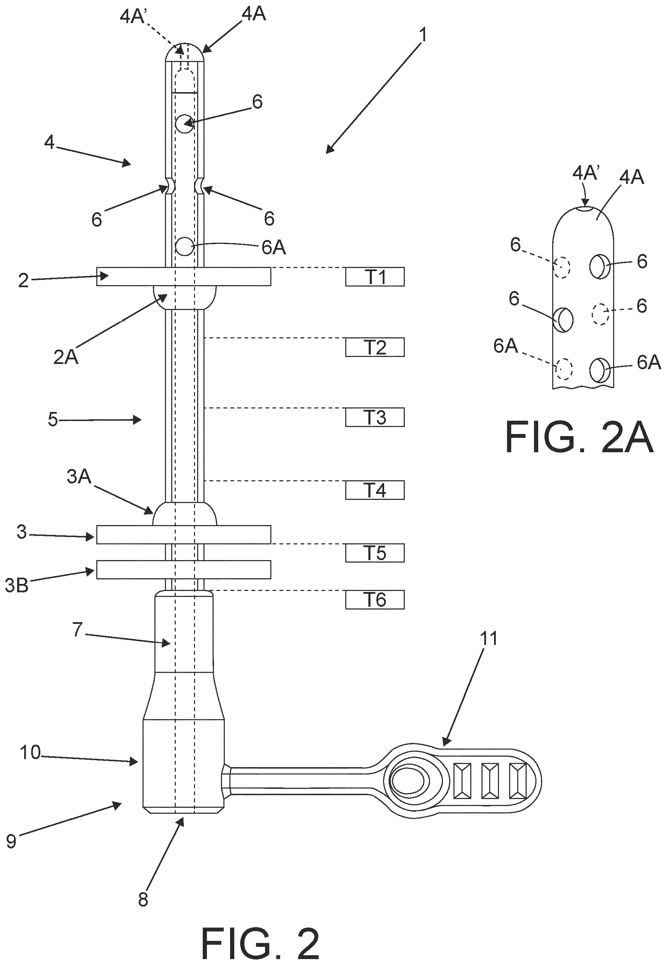

Figure 2 illustrates the catheter 1 shown herein regarding the indications referring to spacing measures between discs 2 and 3, where dashed lines T1, T2, T3, T4, T5 and T6 correspond to 0cm, 1cm, 1.5cm, 2cm, 2.5cm and 3cm measurements, respectively.

Another objective of this permanent, removable and self-sustainable urinary catheter incorporates an external chuck M, in order to maintain the positioning of discs 2, 3 and 3B during the introduction of the urinary catheter 1 through the urethra U of the patient.

The external chuck M comprises a tubular element including a longitudinal opening 12 which extends for the entire length of the external chuck M.

The external chuck M comprises a tubular structure of hardened texture, including a longitudinal opening 12, that envelops the catheter and enables handling without affecting the route, and the external chuck is removed after the end of the procedure.

In male patients, the urinary catheter 1 must be introduced through a minor perineal urethrostomy, whereas in female patients, the introduction of the urinary catheter 1 is performed via the urethra U up to the bladder neck CV.

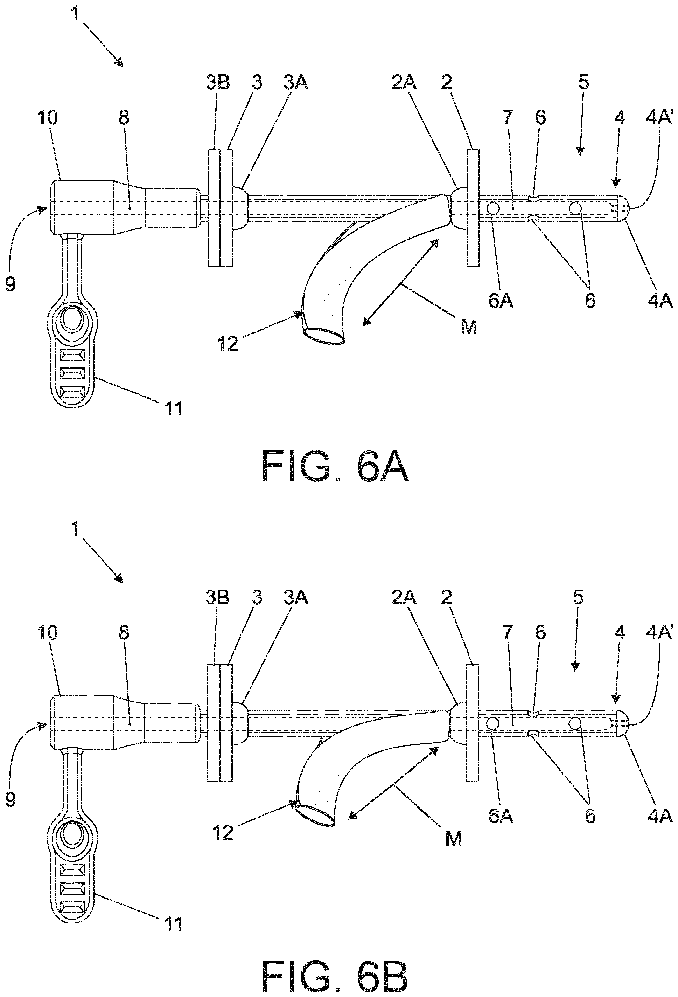

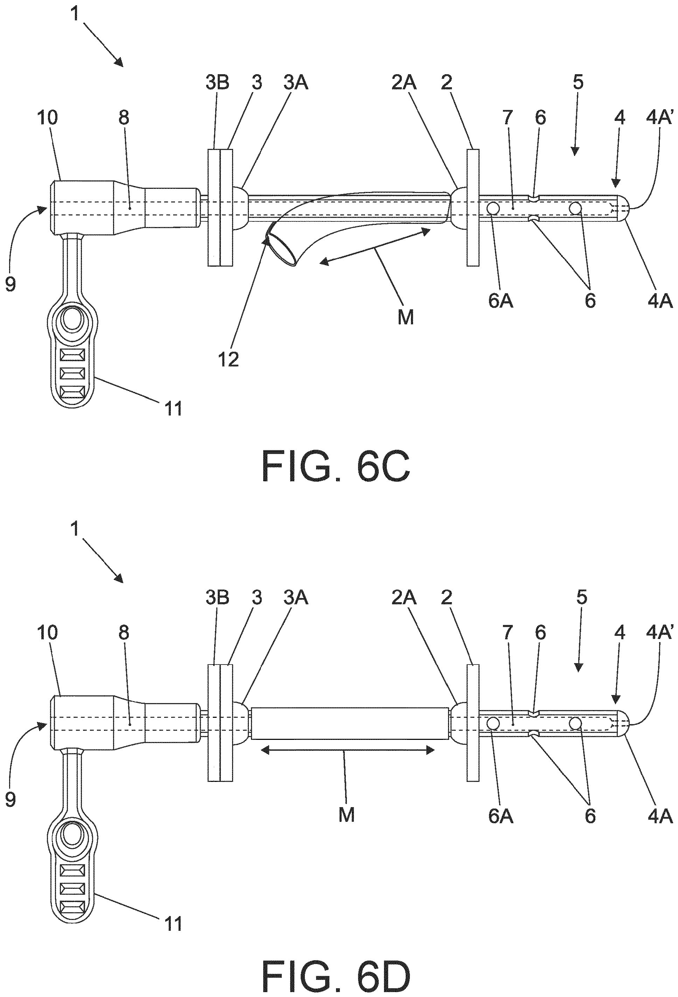

As illustrated by figures 6A to 6D, the external chuck M is installed in the urinary catheter 1 during the production process and is responsible to maintain spacing between discs 2, 3 and 3B.

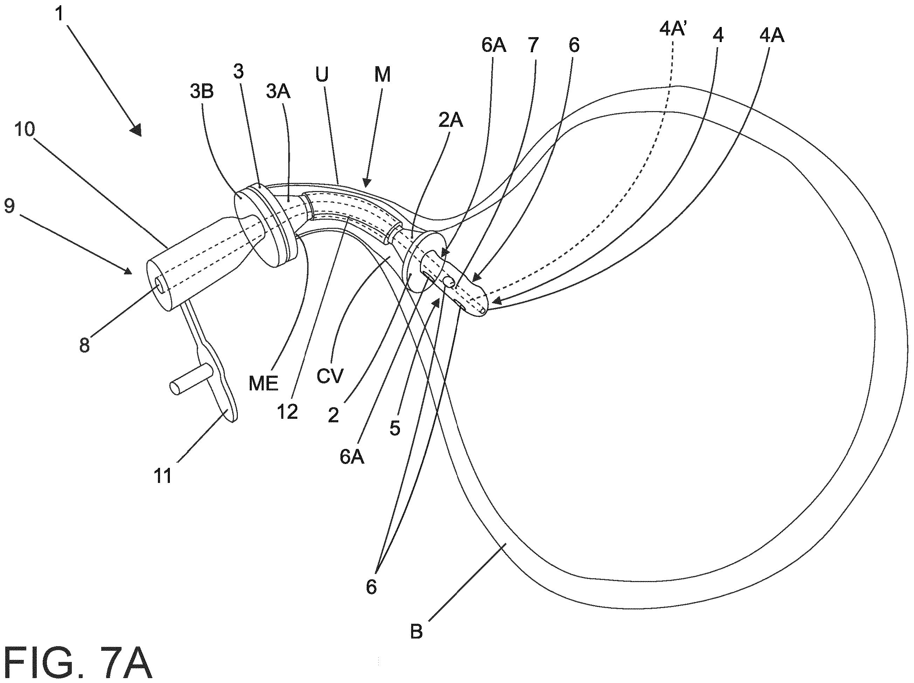

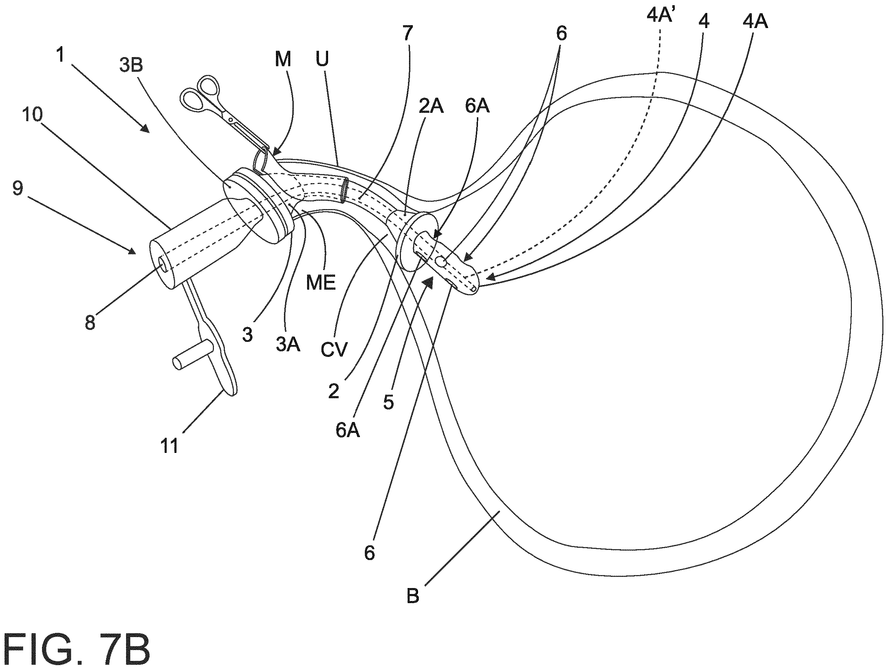

After installation of the urinary catheter 1 in the patient, as illustrated by figure 7A, the physician removes the external chuck , such as illustrated by figure 7B, and carries out the position adjustment of discs 2, 3 and 3B as required, to provide correct tightness and prevent urine leakage.

Whereas the preferred embodiment of this invention patent application is described, any amendments and/or changes must be understood as within the scope of this invention patent application, perfectly conforming to the criteria that define them.

## Claims

1. A permanent, removable and self-sustainable urinary catheter, comprising an urinary catheter (1) having an structure produced in medical grade silicone with three discs, a fixed proximal disc (2) having a central protrusion (2A); and a distal disc (3) having a central protrusion (3A), said distal disc (3) is configured to be slided and adjusted to the female external meatus or male perineal urethrostomy; whereas on the distal end (9) of the catheter (1) there is a connector (10) with a lid (11) and an opening and closing mechanism; a chuck (M) is provided, which features a rod-shaped portion (M1) dimensioned to be introduced along the tubular body (5) of the catheter (1); an enlarged distal portion (M2) matches, when touching the connector end (10), with its end (M3), reaching the final point of the closed proximal end (4) of the urinary catheter (1); and a handle portion (M4), through which the chuck (M) is handled; the tubular body (5) of the catheter (1) including orifices (6, 6A), wherein the tubular body (5) of the urinary catheter (1) includes four orifices (6) and two orifices (6A), the orifices (6A) being closest to the proximal disc (2) and positioned diametrically opposed to each other; the tubular body (5) between proximal disc (2) and distal disc (3) is 2.5 cm long, the catheter (1) has 13 and 15Fr gauges for child use, and 17, 19, 21 and 23Fr for adult use; the full length of the catheter (1) is 7cm for the child model; 8cm for adult model and 15cm for patients with special needs; **characterised in that** the catheter (1) includes a second sliding distal disc (3B).

2. The permanent, removable and self-sustainable urinary catheter, according to claim 1, wherein it further includes an external chuck (M) made of a tubular structure of hardened texture and including a longitudinal opening (12).

## Patentansprüche

1. Ein permanenter, entfernbarer und selbstnachhaltiger Urinkatheter, bestehend aus einem Urinkatheter (1) mit einer Struktur aus medizinischem Silikon, die drei Scheiben aufweist, eine feste proximale Scheibe (2) mit einem zentralen Vorsprung (2A); und eine distale Scheibe (3) mit einem zentralen Vorsprung (3A), sodass die besagte distale Scheibe (3) so konfiguriert ist, dass sie an den weiblichen äußeren Meatus oder die männliche perineale Urethrostomie verschoben und angepasst werden kann; wobei sich am distalen Ende (9) des Katheters (1) ein Anschluss (10) mit einem Deckel (11) und einem Öffnungs- und Schließmechanismus befindet; ein Spannfutter (M) ist vorgesehen, das einen stabförmigen Abschnitt (M1) aufweist, der so dimensioniert ist, dass er entlang des Röhrenkörpers (5) des Katheters (1) eingeführt werden kann; ein verbreiterter distaler Abschnitt (M2) passt, wenn er das Anschlussende (10) berührt, mit seinem Ende (M3) zusammen und erreicht den Endpunkt des geschlossenen proximalen Endes (4) des Urinkatheters (1); und ein Griffteil (M4), durch den das Spannfutter (M) gehandhabt wird; der Röhrenkörper (5) des Katheters (1) umfasst Öffnungen (6, 6A), **dadurch gekennzeichnet, dass** der Röhrenkörper (5) des Urinkatheters (1) vier Öffnungen (6) und zwei Öffnungen (6A) aufweist, wobei sich die Öffnungen (6A) in nächster Nähe zur proximalen Scheibe (2) befinden und einander diametral gegenüberliegen; der Röhrenkörper (5) zwischen der proximalen Scheibe (2) und der distalen Scheibe (3) ist 2,5 cm lang, der Katheter (1) hat die Maßeinheit 13 und 15Fr für Kinder und 17, 19, 21 und 23Fr für Erwachsene; die Gesamtlänge des Katheters (1) beträgt 7 cm für das Kindermodell, 8 cm für das Erwachsenenmodell und 15 cm für Patienten mit besonderen Bedürfnissen; **dadurch gekennzeichnet, dass** der Katheter (1) eine zweite verschiebbare distale Scheibe (3B) umfasst.

2. Der permanente, entfernbarer und selbstnachhaltige Urinkatheter, nach Anspruch 1, wobei er zusätzlich ein externes Spannfutter (M) umfasst, das aus einer röhrenförmigen Struktur mit gehärteter Textur besteht und eine Längsöffnung (12) aufweist.

## Revendications

1. Sonde urinaire permanente, amovible et autonome, comprenant une sonde urinaire (1) présentant une structure fabriquée en silicone de qualité médicale composée de trois disques , un disque proximal fixe (2) présentant une saillie centrale (2A) ; et un disque distal (3) présentant une saillie centrale (3A), ledit disque distal (3) est conçu pour coulisser et s'ajuster au méat externe féminin ou à l'urétrostomie périnéale masculine ; tandis qu'à l'extrémité distale (9) de la sonde (1) se trouve un connecteur (10) muni d'un couvercle (11) et d'un mécanisme d'ouverture et de fermeture ; un mandrin (M) est prévu, lequel présente une partie en forme de tige (M1) dimensionnée pour être introduite le long du corps tubulaire (5) de la sonde (1) ; une partie distale élargie (M2) correspond, lorsqu'elle touche l'extrémité du connecteur (10), à son extrémité (M3), atteignant le point final de l'extrémité proximale fermée (4) de la sonde urinaire (1); et une partie de la poignée (M4) à travers laquelle le mandrin (M) est manipulé; le corps tubulaire (5) de la sonde (1) comprenant des orifices (6, 6A), dans laquelle le corps tubulaire (5) de la sonde urinaire (1) comprend quatre orifices (6) et deux orifices (6A), les orifices (6A) étant les plus proches du disque proximal (2) et positionnés diamétralement opposés l'un à l'autre; le corps tubulaire (5) entre le disque proximal (2) et le disque distal (3) a une longueur de 2,5 cm ; la sonde (1) a des calibres de 13 et 15 Fr pour l'utilisation avec les enfants et de 17, 19, 21 et 23 Fr pour l'utilisation avec les adultes; la longueur totale de la sonde (1) est de 7 cm pour le modèle infantile; 8 cm pour le modèle adulte et 15 cm pour les patients ayant des besoins particuliers; **caractérisé en ce que** la sonde (1) comprend un deuxième disque distal coulissant (3B).

2. Sonde urinaire permanent, amovible et autonome, selon la revendication 1, où elle comprend également un mandrin externe (M) constitué d'une structure tubulaire de texture durcie et comprenant une ouverture longitudinale (12).
